(19) Europäisches Patentamt
European Patent Office
Office européen des brevets

(11) **EP 2 171 525 B1**

(12) **EUROPÄISCHE PATENTSCHRIFT**

(45) Veröffentlichungstag und Bekanntmachung des Hinweises auf die Patenterteilung:
**03.04.2019 Patentblatt 2019/14**

(21) Anmeldenummer: **08773985.0**

(22) Anmeldetag: **14.07.2008**

(51) Int Cl.:
*G02C 7/02* (2006.01)    *A61B 3/02* (2006.01)
*A61B 3/10* (2006.01)

(86) Internationale Anmeldenummer:
**PCT/EP2008/005733**

(87) Internationale Veröffentlichungsnummer:
**WO 2009/007136 (15.01.2009 Gazette 2009/03)**

(54) **VERFAHREN ZUM ÜBERPRÜFEN UND/ODER BESTIMMEN VON BENUTZERDATEN, COMPUTERPROGRAMMPRODUKT UND VORRICHTUNG**

METHOD FOR CHECKING AND/OR DETERMINING USER DATA, COMPUTER PROGRAM PRODUCT, AND DEVICE

PROCÉDÉ DE CONTRÔLE ET/OU DE DÉTERMINATION DE DONNÉES D'UTILISATEUR, PRODUIT-PROGRAMME INFORMATIQUE ET DISPOSITIF

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.07.2007 DE 102007032564**

(43) Veröffentlichungstag der Anmeldung:
**07.04.2010 Patentblatt 2010/14**

(73) Patentinhaber: **Rodenstock GmbH**
**80687 München (DE)**

(72) Erfinder:
- **ESSER, Gregor**
  **81735 München (DE)**
- **SEIDEMANN, Anne**
  **80337 München (DE)**
- **BECKEN, Wolfgang**
  **81541 München (DE)**
- **WEHNER, Edda**
  **82275 Emmering (DE)**
- **ALTHEIMER, Helmut**
  **87650 Baisweil-Lauchdorf (DE)**
- **MÜLLER, Werner**
  **75443 Ötisheim (DE)**
- **UTTENWEILER, Dietmar**
  **82057 Icking (DE)**

(74) Vertreter: **Müller-Boré & Partner**
**Patentanwälte PartG mbB**
**Friedenheimer Brücke 21**
**80639 München (DE)**

(56) Entgegenhaltungen:
WO-A-2005/058136     US-A1- 2002 140 902
US-A1- 2005 225 721     US-A1- 2005 280 777

- THIBOS L N ET AL: "POWER VECTOR: AN APPLICATION OR FOURIER ANALYSIS TO THE DESCRIPTION AND STATISTICAL ANALYSIS OF REFRACTIVE ERROR" OPTOMETRY AND VISION SCIENCE, WILLIAMS AND WILKINS, BALTIMORE, MD, US, Bd. 74, 1. Januar 1977 (1977-01-01), Seiten 367-375, XP008078911 ISSN: 1040-5488
- CHOI M ET AL: "LABORATORY, CLINICAL, AND KINDERGARTEN TEST OF A NEW ECCENTRIC INFRARED PHOTOREFRACTOR (POWERREFRACTOR)", OPTOMETRY AND VISION SCIENCE, WILLIAMS AND WILKINS, BALTIMORE, MD, US, vol. 77, no. 10, 1 October 2000 (2000-10-01), pages 537-548, XP008020388, ISSN: 1040-5488, DOI: 10.1097/00006324-200010000-00008
- KUSEL R ET AL: "Light-intensity distribution in eccentric photorefraction crescents", 1 June 1998 (1998-06-01), JOURNAL OF VACUUM SCIENCE AND TECHNOLOGY: PART B, AVS / AIP, MELVILLE, NEW YORK, NY, US, PAGE(S) 1500 - 1511, XP002250296, ISSN: 1071-1023

**Beschreibung**

Die vorliegende Erfindung betrifft Verfahren zum Überprüfen und/oder Bestimmen von Benutzerdaten eines Brillenglasträgers, ein Computerprogrammprodukt und Vorrichtungen zum Überprüfen und/oder Bestimmen von Benutzerdaten des Brillenglasträgers.

Eine weitverbreitete Methode zum Bestimmen einer Refraktion ist die sogenannte subjektive Refraktionsbestimmung, die sich bei Optikern allgemein durchgesetzt hat. Bei der subjektiven Refraktionsbestimmung werden herkömmlicherweise dem Benutzer eines Brillenglases unterschiedliche Brillengläser, d.h. Brillengläser mit unterschiedlichen optischen Eigenschaften vorgesetzt, wobei der Benutzer des Brillenglases dem Refraktionisten eine Verbesserung bzw. eine Verschlechterung des Seheindrucks bei Änderung der optischen Eigenschaften des vorgesetzten Brillenglases mitteilt. Die subjektive Refraktionsbestimmung kann beispielsweise auf Werten einer objektiven Refraktionsbestimmung oder auf Werten einer bereits getragenen Brille aufbauen. Die Genauigkeit der subjektiven Refraktionsbestimmung hängt jedoch kritisch vom Können des Refraktionisten, beispielsweise eines Augenoptikers und/oder eines Augenarztes ab, der die subjektive Refraktionsbestimmung durchführt. Ebenso hängt die subjektive Refraktionsbestimmung kritisch von der zu untersuchenden Person ab, insbesondere von der Fähigkeit der zu untersuchenden Person, die Schärfe der Seheindrücke zu beurteilen und/oder zu artikulieren.

Vielfach unterscheiden sich Meßmethoden zur Bestimmung der subjektiven Refraktion je nach Augenoptik-Hochschule mitunter stark und sind auch international verschieden. Dadurch ergeben sich größere Unterschiede in der Ergebnissen.

Da weiterhin die einzelnen Refraktionsgläser maximal nur in etwa 0,125 dpt, in der Regel nur in etwa 0,25 dpt Abstufungen zur Verfügung stehen, liegt die maximale (theoretische) Genauigkeit in der Regel auch nur bei 0,125 dpt.

Außerdem wird die subjektive Refraktion nur bei einer Kontraststärke, in der Regel bei maximalem Kontrast und kleinster Auflösung, durchgeführt. Ob die festgestellten Refraktionswerte auch die idealen Refraktionswerte bei schlechtem Kontrast und geringer Auflösung darstellen, wird nicht überprüft.

Die Refraktionsbestimmung wird nur bei einer Pupillengröße durchgeführt, in der Regel bei hoher Beleuchtung und kleiner Pupille. Es wird nicht überprüft, ob die Refraktion mit der Pupillengröße variiert.

Ferner können die Umgebungsparameter je nach Prüfort mitunter sehr stark variieren und haben einen erheblichen, unkontrollierten Einfluß auf das Ergebnis. Die Umgebungsparameter stellen beispielsweise Kontrast der Sehprobe, Entfernung der Sehprobe, Größe der Sehzeichen, Form der Sehzeichen und Raum-Helligkeit dar. Die subjektive Refraktion liefert daher häufig nur wenig zufriedenstellende Ergebnisse.

US 2002/0140902 A1 offenbart ein Verfahren und eine Vorrichtung zum Bestimmen von Individualparametern zur Herstellung eines Brillenglases unter Verwendung einer Aberrationsmessung am Auge des Brillenträgers. Ausgehend von den Aberrationsmessungen wird eine Metrik bestimmt und die Metrik mit gemessenen subjektiven Refraktionsdaten verglichen. Die Metrik wird verwendet, um die Parameter eines Brillenglases zu berechnen, das zur Korrektur der Fehlsichtigkeit des Auges des Brillenträgers verwendet wird.

WO 2005/058136 A2 offenbart, daß basierend auf Refraktionsdaten in Verbindung mit Patientendaten, Umgebungsdaten und klinischen Überlegungen die Verordnung eines Patienten bestimmt, insbesondere berechnet wird. Es ist daher eine Aufgabe der vorliegenden Erfindung, in einfacher Weise genaue Refraktionsdaten eines Benutzers eines Brillenglases zur Verfügung zu stellen.

Diese Aufgabe wird gelöst anhand der Gegenstände der unabhängigen Ansprüche. Bevorzugte Ausführungsvarianten bzw. -formen sind Gegenstand der abhängigen Ansprüche.

Verfahren gemäß eines Aspekts der Erfindung

Ein Aspekt der vorliegenden Erfindung betrifft ein Verfahren zum Herstellen eines Brillenglases mit den Merkmalen des Anspruchs 1.

Vorteilhafterweise werden somit die subjektive Refraktionsmessung und die objektive Refraktionsmessung miteinander verbunden. Beispielsweise können aufgrund von Adaptionen des visuellen Systems Abweichungen zwischen der subjektiven und der objektiven Refraktion auftreten. Diese Abweichungen können in einfacher und sicherer Weise festgestellt und gegebenenfalls ausgeglichen werden. Insbesondere kann vorteilhafterweise überprüft werden, ob die ermittelten Refraktionswerte für die zukünftigen Benutzer physiologisch verträglich sind. Beispielsweise tritt es häufig auf, daß die "perfekte" Abbildung, d.h. eine Abbildung gemäß objektiver Refraktionsdaten, subjektiv nicht als die beste empfunden wird, da das visuelle System sensorisch schon an die Fehlrefraktion adaptiert ist. Weist beispielsweise der Benutzer eine leichte Hyperopie auf, so kann der Benutzer diese Hyperopie, solange er noch nicht presbyop ist, durch Akkommodation ausgleichen. Wird beispielsweise eine solche Hyperopie anhand der objektiven Refraktionsdaten vollständig auskorrigiert, kann dies zu Unverträglichkeiten führen. Gleiches gilt für astigmatische Fehlsichtigkeiten. Hierbei kann jedoch der Unterschied zwischen subjektiver und objektiver Refraktion noch gravierender sein. So kann beispielsweise eine Vollkorrektion gemäß objektiver Refraktionsdaten subjektiv nicht als beste Korrektion wahrgenommen werden. Gemäß der vorliegenden Erfindung ist es jedoch möglich, beispielsweise (gravierende) Abweichungen der subjektiven Refraktionsdaten von den objektiven Refraktionsdaten festzustellen und hierbei beispielsweise festzustellen, ob der Refraktionist eine fehlerhafte Refraktionsbestimmung durchgeführt hat. Gegebenenfalls können die subjektiven Refraktionsdaten zumindest teilweise an die objektiven Refraktionsdaten angepaßt werden. Zusätzlich oder alternativ können zumindest für einen Teil der Refraktionsdaten die anhand der subjektiven Refraktionsmessung erhaltenen Werte beibehalten werden, auch wenn diese Werte von den objektiven Refraktionsdaten abweichen. Insbesondere kann eine Vergleichsbedingung festgelegt werden, wobei keine Anpassung der subjektiven Refraktionsdaten vorgenommen wird, sofern der Unterschied zwischen den objektiven Refraktionsdaten und den subjektiven Refraktionsdaten geringer ist, als die Vergleichsbedingung. Als Beispiel kann eine maximale Abweichung der sphärischen Brechkraft, des Astigmatismus, insbesondere der Achslage des Astigmatismus, usw. angegeben bzw. vorgegeben werden und, sofern die Abweichung geringer als die maximale, vorgegebene Abweichung ist, keine Anpassung der subjektiven Refraktionsdaten vorgenommen werden. Als weiteres Beispiel kann ein maximaler Wert der Sphäre und/oder des Zylinders der nach der Kreuzzylindermethode berechneten Adweichung zwischen den subjektiven und den objektiven Refraktionsdaten vorgegeben werden. In beiden Fällen kann die maximal erlaubte Abweichung bspw. 0,1 dpt, 0,2 dpt, 0,5 dpt, 1 dpt, 2 dpt, usw. sein. In anderen Worten kann die Vergleichsbedingung der maximalen Abweichung entsprechen. Die maximale Abweichung kann auch ein relativer Wert, beispielsweise in Prozent sein. Die maximale Abweichung, d.h. die Vergleichsbedingung kann ein Unterschied zwischen einem objektiven und einem subjektiven Wert (bzw. mehreren solchen Werten) zwischen etwa 1% und etwa 50%, insbesondere zwischen etwa 10% und etwa 30%, z.B. auch eine Abweichung um etwa 1%, 2%, 3%, 5%, 10%, 15%, 20%, 25%, usw. sein.

Alternativ oder zusätzlich kann auch eine Nachricht ausgegeben werden, die das Vergleichsergebnis enthält. Insbesondere kann die Nachricht eine Warnnachricht sein, welche darauf hinweist, daß beispielsweise eine Teilmenge der subjektiven Refraktionsdaten und eine Teilmenge der objektiven Refraktionsdaten eine vorbestimmte Vergleichsbedingung nicht erfüllen, d.h. daß eine Abweichung zumindest einer Teilmenge der subjektiven Refraktionsdaten von den entsprechenden objektiven Refraktionsdaten vorliegt, die beispielsweise größer als eine vorbestimmte Vergleichsbedingung ist. In einem nächsten Schritt kann beispielsweise der Refraktionist die subjektiven Refraktionsdaten manuell ändern, insbesondere an die objektiven Refraktionsdaten anpassen. Der Refraktionist kann auch die subjektive Refraktionsbestimmung zumindest teilweise wiederholen. Es ist auch möglich, daß die subjektiven Refraktionsdaten automatisch an die objektiven Refraktionsdaten angepaßt werden.

Der Begriff "Bereitstellen" im Sinne der vorliegenden Erfindung beinhaltet "Messen", "Schätzen", "Übermitteln", "Entnehmen einer Datenbank und/oder einer Tabelle", "Senden", usw.

Die subjektiven Daten enthalten zumindest subjektive Refraktionsdaten. Die subjektiven Daten können auch weitere Daten enthalten, beispielsweise das Alter des Brillenglasbenutzers, eine Entwicklung der Fehlsichtigkeit des Brillenglasbenutzers, eine Krankheitshistorie des Brillenglasbenutzers, usw.

Die subjektiven Daten können beispielsweise von einem Optiker, Augenarzt usw. aufgenommen werden. Die subjektiven Daten können auch in einer lokalen oder einer in einem Netzwerk eingebundenen Datenbank oder einer Server-gestützten Datenbank oder einer externen Datenbank usw. bereitliegen. Diese Daten können um die subjektiven Refraktionsdaten ergänzt werden bzw. die subjektiven Refraktionsdaten nach Refraktionsbestimmung erneuert werden. Beispielsweise können die subjektiven Daten an einem Brillenglashersteller übermittelt werden. Diese Übermittlung kann beispielsweise auf dem Postweg erfolgen. Die subjektiven Daten können auch über ein Netzwerk, beispielsweise das Internet, usw. übermittelt werden.

Ebenso können die objektiven Refraktionsdaten einer Datenbank entnommen werden bzw. von dem Augenoptiker, dem Augenarzt, usw. aktualisiert werden. Die objektiven Refraktionsdaten können (anschließend) an einen Brillenglashersteller übermittelt werden.

Die objektiven Refraktionsdaten und/oder zumindest eine Teilmenge der subjektiven Daten können auch bei dem Brillenglashersteller hinterlegt sein.

Weiterhin kann der Vergleich zumindest der Teilmenge der subjektiven Refraktionsdaten mit zumindest der Teilmenge der objektiven Refraktionsdaten beim Augenoptiker, Augenarzt, usw. vorgenommen werden, und ebenso können dort Vergleichsergebnisse ausgegeben werden bzw. zumindest eine Teilmenge der zugegebenen Refraktionsdaten gegebenenfalls angepaßt werden. Alternativ oder zusätzlich kann der Vergleich, gegebenenfalls das Anpassen und/oder das Ausgeben der Nachricht, auch beim Hersteller des Brillenglases stattfinden.

Anpassen zumindest der Teilmenge der subjektiven Refraktionsdaten an die objektiven Refraktionsdaten kann insbesondere beinhalten, daß ein oder mehrere Werte der subjektiven Refraktionsdaten geändert werden. Beispielsweise kann ein Wert der subjektiven Refraktionsdaten, der von einem anhand einer objektiven Refraktion gemessenen Wert abweicht, an den durch die objektive Messung bestimmten Wert angeglichen werden. Der Wert der subjektiven Refraktionsmessung kann z.B. dem Wert der objektiven Refraktionsmessung gleichgesetzt werden. Der Wert der subjektiven Refraktionsmessung kann auch dem Mittelwert zwischen dem subjektiven und dem objektiven Refraktionswert gleichgesetzt werden. Es ist auch möglich, daß der subjektive Refraktionswert um 5%, 10% oder 15%, 20% usw. der Differenz des Wertes der subjektiven Refraktionsmessung und der objektiven Refraktionsmessung vergrößert oder verkleinert wird.

Anhand der Kombination der subjektiven Refraktionsdaten und der objektiven Refraktionsdaten, insbesondere anhand der möglichen selektiven Änderung der subjektiven Refraktionsdaten und/oder Beibehalten der subjektiven Refraktionsdaten, auch bei Abweichung von den objektiven Refraktionsdaten, kann vorteilhafterweise eine gewollte Abweichung der subjektiven von den objektiven Refraktionsdaten insbesondere aufgrund von geometrisch-optischen Abbildungsdifferenzen beibehalten werden. Solche Abbildungsdifferenzen können aufgrund des Abstandes Brillenglas-Auge insbesondere bei einer Korrektion von astigmatischen Fehlsichtigkeiten auftreten, da eine ungewohnte Verzeichnung durch den Abstand Brillenglas Auge vorliegen kann. Da auch in diesem Fall oftmals eine Vollkorrektion gemäß objektiver Refraktionsdaten subjektiv nicht als die beste Korrektion empfunden wird, kann es vorteilhaft sein, die subjektiven Refraktionsdaten nicht bzw. nur eine Teilmenge der subjektiven Refraktionsdaten an die objektiven Refraktionsdaten anzupassen. Es kann auch nur eine geringfügige Anpassung beispielsweise um wenige Prozentpunkte (1%, 2%, 3%, 5%, 10%, usw.) der subjektiven Refraktionsdaten an die objektiven Refraktionsdaten vorgenommen werden. Somit kann vorteilhafterweise der Einfluß von geometrisch-optischen Abbildungsdifferenzen berücksichtigt werden.

Weiterhin vorteilhafterweise kann anhand der Kombination von subjektiver und objektiver Refraktionsmessung ein Binokularsehen berücksichtigt werden, welches insbesondere bei herkömmlichen objektiven Meßverfahren vernachlässigt ist. Hierbei hat aufgrund der starken Kopplung von Akkommodation und Konvergenz des Binokularsehen sehr großen Einfluß auf die Refraktionswerte. Bei einer Exophorie, bei Divergenz-Exzess und/oder bei Konvergenz-Insuffizienz treten oftmals große Unterschiede zwischen den monokularen und binokularen Refraktionswerten auf. Beispielsweise verschieben sich Binokularwerte Richtung Minus, da mit der damit benötigten Akkommodation und daraus resultierenden Konvergenz die Exophorie, d.h. einem zweiteiligen Abweichen der Sehachse nach außen, ausgeglichen wird. Umgekehrt verschieben sich die Werte bei einer Esophorie, Divergenz-Insuffizienz oder Konvergenz-Exzess, d.h. Einwärtsschielen, oftmals Richtung Plus. Bei einer subjektiven Refraktionsbestimmung können diese Einflüsse berücksichtigt werden, da insbesondere die subjektive Refraktionsbestimmung binokular möglich ist. Durch entsprechendes Anpassen der Vergleichsbedingung können die subjektiven Refraktionsdaten und die objektiven Refraktionsdaten miteinander verglichen werden und beispielsweise eine Abweichung der subjektiven Refraktionsdaten von objektiven Refraktionsdaten aufgrund der binokularen subjektiven Refraktionsmessung erlaubt sein.

Weiterhin vorteilhafterweise kann insbesondere aufgrund eines schematischen Vorgehens bei einer subjektiven Refraktionsbestimmung eine Akkommodation kontrolliert bzw. berücksichtigt werden. Der Einfluß der Akkommodation auf eine objektive Refraktionsmessung ist häufig schwierig zu bestimmen bzw. kann nicht berücksichtigt werden.

Bevorzugte Ausführungsvarianten des Verfahrens

Vorzugsweise umfaßt das Verfahren ein Bereitstellen einer Nachricht, welche das Vergleichsergebnis enthält.

Gemäß einer bevorzugten Ausführungsvariante werden Sollwerte zumindest eines Brillenglases anhand folgender Daten bestimmt:

- Zumindest eine Teilmenge der subjektiven Refraktionsdaten und/oder
- zumindest einer Teilmenge der angepaßten subjektiven Refraktionsdaten und/oder
- zumindest einer Teilmenge der objektiven Refraktionsdaten.

Der Begriff "Bestimmen" im Sinne der vorliegenden Anmeldungen beinhaltet ein Berechnen oder ein Abschätzen zumindest eines Sollwerts. Beispielsweise kann anhand der vorgegebenen Daten ein Sollwert einer Datenbank entnommen werden und/oder anhand vorgegebener Optimierungsalgorithmen berechnet werden. Hierbei können beispielsweise ein oder mehrere Optimierungsverfahren verwendet werden.

Der Begriff "Sollwert" im Sinne der vorliegenden Anmeldung beinhaltet einen gewünschten Wert eines Brillenglases. In anderen Worten ist der Sollwert des Brillenglases der Wert, der den Vorgaben durch einen Augenoptiker, einen Augenarzt und/oder einen Hersteller entspricht. Der Sollwert kann mehrere Werte umfassen. Insbesondere kann der Sollwert Werte für sphärische Wirkung, Zylinder, Achse etc. beinhalten, die das Brillenglas aufweisen soll. Bei einem idealen Brillenglas entsprechen die tatsächlichen optischen Eigenschaften dem Sollwert.

Weiterhin vorzugsweise werden die subjektiven Refraktionsdaten und/oder die angepaßten subjektiven Refraktionsdaten und/oder die objektiven Refraktionsdaten zu kombinierten Refraktionsdaten kombiniert.

Die subjektiven Refraktionsdaten können hierbei die durch subjektive Refraktionsmessung bereitgestellten Refraktionsdaten sein. Die angepaßten subjektiven Refraktionsdaten können beispielsweise die anhand der objektiven Refraktionsdaten angepaßten subjektiven Refraktionsdaten sein bzw. eine Teilmenge dieser subjektiven Refraktionsdaten. Die objektiven Refraktionsdaten sind die anhand eines objektiven Verfahrens bereitgestellten objektiven Refraktionsdaten.

Insbesondere kann eine Teilmenge oder eine Gesamtmenge der vorgenannten Daten verwendet werden. Beispielsweise können Sollwerte anhand von kombinierten objektiven Refraktionsdaten, die durch Kombination von

- zumindest einer Teilmenge der subjektiven Refraktionsdaten und/oder
- zumindest einer Teilmenge angepaßten subjektiven Refraktionsdaten und/oder
- zumindest einer Teilmenge der objektiven Refraktionsdaten gebildet werden,

bestimmt werden.

Weiterhin vorzugsweise werden effektive objektive Refraktionsdaten anhand zumindest einer Teilmenge der objektiven Refraktionsdaten hergestellt, wobei zum Bestimmen der Vergleichsergebnisse die effektiven objektiven Refraktionsdaten mit zumindest der Teilmenge der subjektiven Refraktionsdaten verglichen werden. Beispielsweise kann eine Vielzahl objektiver Refraktionsdaten unter anderem für eine Vielzahl von Pupillengrößen, unterschiedlichen Randbedingungen, wie Helligkeit, Kontrast, usw. erstellt werden und anhand dieser Vielzahl objektiver Refraktionsdaten die effektiven objektiven Refraktionsdaten erstellt bzw. bestimmt werden. Beispielsweise ist es möglich, daß lediglich ein Teil der objektiven Refraktionsdaten zu effektiven objektiven Refraktionsdaten verändert wird. Der Rest der objektiven Refraktionsdaten bleibt unverändert. Die Gesamtmenge dieser effektiven objektiven Refraktionsdaten und der verbleibenden objektiven Refraktionsdaten kann als effektive objektive Refraktionsdaten bezeichnet werden. Diese effektiven objektiven Refraktionsdaten können mit den subjektiven Refraktionsdaten verglichen werden.

Beispielsweise ist es möglich, Sollwerte anhand von kombinierten objektiven Refraktionsdaten, die durch Kombination von

- zumindest einer Teilmenge der subjektiven Refraktionsdaten und/oder
- zumindest einer Teilmenge einer angepaßten subjektiven Refraktionsdaten und/oder
- zumindest einer Teilmenge der effektiven objektiven Refraktionsdaten gebildet werden,

zu bestimmen.

Gemäß einer weiteren bevorzugten Ausführungsform werden mehrere Sätze objektiver Refraktionsdaten bereitgestellt und die effektiven objektiven Refraktionsdaten anhand der mehreren Sätze objektiver Refraktionsdaten erstellt. Die Sätze objektiver Refraktionsdaten können beispielsweise für unterschiedliche Randbedingungen (siehe oben) erstellt werden.

Beispielsweise werden die effektiven objektiven Refraktionsdaten unter Beachtung folgender Randbedingungen bestimmt:

- Helligkeit und/oder
- Pupillendurchmesser und/oder
- Kontrast und/oder
- Alter.

Vorzugsweise werden zur Berechnung der effektiven objektiven Refraktionsdaten eine Aberrationsfunktion (engl.: aberation function oder wave aberration) und/oder eine optische Transferfunktion (bezeichnet als "OTF") und/oder eine Modulationsübertragungsfunktion (bezeichnet als "MTF") und/oder eine sogenannte point spread function (bezeichnet als "PSF") und/oder eine Standardabweichung und/oder ein Strehl Verhältnis berechnet. In diesem Zusammenhang wird auf die einschlägige Literatur verwiesen. Konkrete Definitionen der Aberrationsfunction finden sich beispielsweise in dem Buch "Principles of Optics" von Born und Wolf, Pergamon Press (1980), auf Seiten 203 ff. und Seiten 459 ff. Eine Definition des Strehlverhältnisses findet sich beispielsweise auf Seiten 462 ff. Definitionen der "OTF" und der "MTF" sowie eine Diskussion davon sind beispielsweise in dem Buch "Introduction to Fourier Optics" von Goodman, McGraw-Hill, 1968, enthalten. Die point spread function ("PSF") ist die Fouriertransformierte der "OTF" und ist die Punktbildfunktion, d.h. beschreibt die Intensitätsverteilung des Bildes, das ein optisches System von einer punktförmigen Lichtquelle entwirft. Die PSF ist zum Beispiel in dem Buch "The eye and visual optical instruments", von Smith/Atchison, Cambridge University Press, 1996, definiert und erläutert.

Besonders bevorzugt wird eine Aberrationsfunktion berücksichtigt, wobei die berücksichtigte Ordnung der Aberration variabel ist.

In anderen Worten kann beispielsweise die Ordnung der Aberration einmal gewählt werden und anschließend beibehalten werden. Es ist auch möglich, daß die Ordnung der Aberration kontinuierlich bzw. stufenweise bzw. iterativ, usw. verändert wird.

Gemäß einer weiteren bevorzugten Ausführungsform wird zumindest eine Teilmenge der objektiven Refraktionsdaten anhand exzentrischer Photorefraktion bestimmt.

Insbesondere kann zumindest die Teilmenge der objektiven Refraktionsdaten monokular oder binokular bestimmt werden.

Computerprogrammprodukt gemäß eines Aspekts der Erfindung

Ein weiterer Aspekt der vorliegenden Erfindung betrifft ein Computerprogrammprodukt, insbesondere auf einem computerlesbaren Medium gespeichert oder als Signal verwirklicht, welches, wenn geladen in den Speicher eines Computers und ausgeführt von einem Computer, bewirkt, daß der Computer ein erfindungsgemäßes Verfahren durchführt.

Vorrichtung gemäß eines Aspekts der Erfindung

Ein weiterer Aspekt der vorliegenden Erfindung betrifft eine Vorrichtung zum Herstellen eines Brillenglases mit den Merkmalen des Anspruchs 11.

Bevorzugte Ausführungsformen der Vorrichtungen

Vorzugsweise gelten die vorgenannten Ausführungen und Vorteile zu den Verfahren sinngemäß auch für die vorgenannten Vorrichtungen. Insbesondere weisen die vorgenannten Vorrichtungen Mittel bzw. Einrichtungen auf, die ausgelegt sind, die vorgenannten Verfahrensschritte durchzuführen.

Figurenbeschreibung

Bevorzugte Ausführungsvarianten bzw. bevorzugte Ausführungsformen der vorliegenden Erfindung werden nachfolgend anhand begleitender Figuren beispielhaft beschrieben. Es zeigt,

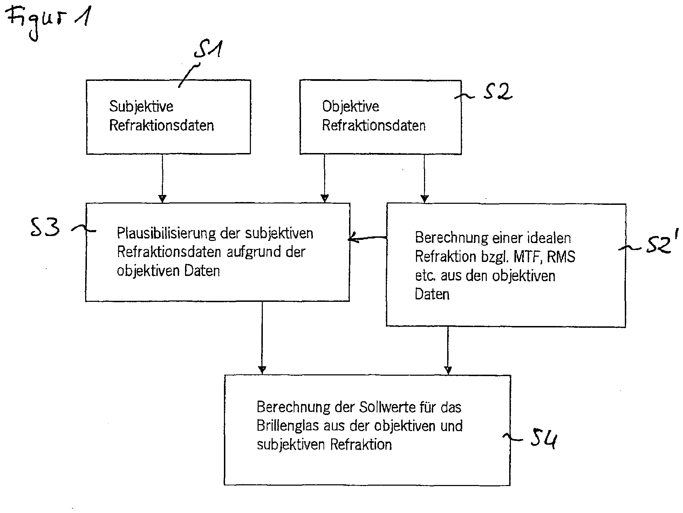

Figur 1:  ein Flußdiagramm.

**Figur 1** beschreibt beispielhafte Verfahren zur Überprüfung von Benutzerdaten eines Brillenglasträgers bzw. zur Herstellung eines Brillenglases. In Schritt S1 werden subjektive Daten des Benutzers bestimmt, d.h. die subjektiven Daten werden bereitgestellt. Insbesondere werden in Schritt 1 subjektive Refraktionsdaten bestimmt. Die subjektiven Daten enthalten beispielsweise auch Informationen über das Alter, die Entwicklung der Fehlsichtigkeit des Benutzers, das Geschlecht des Benutzers, Berufsausübung des Benutzers, eine Krankheitsgeschichte, usw. Die subjektiven Refraktionsdaten beinhalten insbesondere ein oder mehrere herkömmliche Refraktionsdaten, wie beispielsweise Sphäre, Zylinder, Achslage, usw.
Die subjektiven Daten können auch individuelle Parameter des Benutzers beinhalten, wie zum Beispiel Pupillendistanz, Hornhautscheitelabstand, Fassungsscheibenvorneigung, usw. Die subjektiven Refraktionsdaten können beispielsweise bei einem Optiker gemessen werden. Ebenso können weitere subjektive Daten bei einem Optiker gemessen werden bzw. vom Benutzer dem Optiker angegeben werden. Alternativ kann dies auch beim Augenarzt stattfinden. Beispielsweise ist es auch möglich, daß ein Teil der subjektiven Daten beim Augenarzt und ein Teil der subjektiven Daten bei einem Optiker vorliegen. Die Daten können zwischen Augenarzt und Optiker ausgetauscht werden. Zusätzlich oder alternativ können die Daten auch von dem Augenarzt und/oder dem Optiker an einen Hersteller des Brillenglases übermittelt werden. Die Übermittlung kann insbesondere postalisch erfolgen. Die Übermittlung kann aber auch über ein Telefaxgerät, das Internet, anhand eines Speichermediums, usw. erfolgen. Beispielsweise kann der Augenarzt dem Hersteller des Brillenglases eine Teilmenge der subjektiven Daten postalisch zur Verfügung stellen. Der Optiker kann einen Teil der subjektiven Daten dem Hersteller per E-Mail übermitteln. Gegebenenfalls kann der Benutzer des Brillenglases einen Teil der subjektiven Daten dem Optiker zur Verfügung stellen bzw. dem Augenarzt zur Verfügung stellen, insbesondere können diese Daten auf einem Speicherchip, insbesondere einer digitalen/ elektronischen Krankenkarte etc. gespeichert sein.
In einem weiteren Schritt S2 werden objektive Refraktionsdaten bereitgestellt. Die objektiven Refraktionsdaten können beispielsweise durch eine oder mehrere objektive Refraktionsmessungen bei einem Augenarzt oder bei einem Optiker gemessen werden bzw. zwischen Augenarzt und Optiker ausgetauscht werden. Die objektiven Refraktionsdaten können auch dem Hersteller des Brillenglases übermittelt werden.
In einem Schritt S3 kann eine Plausibilisierung des subjektiven Refraktionsdaten aufgrund der objektiven Refraktionsdaten durchgeführt werden. In anderen Worten können die subjektiven Refraktionsdaten und die objektiven Refraktionsdaten miteinander verglichen werden und Abweichungen der subjektiven und der objektiven Refraktionsdaten voneinander bestimmt werden. Diese Plausibilisierung kann z.B. beim Augenarzt, beim Optiker oder auch beim Hersteller durchgeführt werden. Beispielsweise kann ein Teil der Daten beim Augenarzt und/oder beim Optiker plausibilisiert werden. Einen Teil der Daten kann auch beim Hersteller plausibilisiert werden. Es ist auch möglich, daß die Daten sowohl beim Augenarzt als auch beim Optiker als auch beim Hersteller plausibilisiert werden.
In einem weiteren Schritt S4 können anschließend Sollwerte für das Brillenglas aus den objektiven und den subjektiven Refraktionsdaten hergestellt werden. Insbesondere kann es beispielsweise möglich sein, lediglich die subjektiven Refraktionsdaten zur Bestimmung der Sollwerte des Brillenglases zu verwenden, nachdem die subjektiven Refraktionsdaten anhand der objektiven Refraktionsdaten auf Plausibilität geprüft werden. Alternativ können auch die subjektiven Refraktionsdaten und die objektiven Refraktionsdaten in die Bestimmung der Sollwerte eingehen, beispielsweise können subjektive Refraktionswerte bzw. -daten anhand der objektiven Refraktionswerte korrigiert werden. Es ist auch möglich, daß eine Teilmenge der subjektiven Refraktionswerte und eine Teilmange der objektiven Refraktionswerte herangezogen wird, um die Sollwerte des Brillenglases zu berechnen, insbesondere zu bestimmen bzw. ein entsprechendes Brillenglas zu optimieren. Die Sollwerte können beim Augenarzt und/oder beim Optiker und/oder beim Hersteller anhand der subjektiven Refraktionswerte und/oder der objektiven Refraktionswerte bestimmt werden. Werden die Sollwerte nicht beim Hersteller bestimmt, werden in einem weiteren Schritt die Sollwerte dem Hersteller übergeben. Es ist auch möglich, daß, zusätzlich zu den Sollwerten, der Hersteller auch die subjektiven und/oder objektiven Refraktionswerte erhält und somit auch die Sollwerte von dem Hersteller überprüft werden können bzw. der Hersteller eigene Sollwerte bestimmen kann.

Weiterhin vorzugsweise kann in einem Schritt S2' eine ideale Refraktion bezüglich MTF und/oder RMS, usw. aus den objektiven Refraktionsdaten bestimmt, insbesondere berechnet werden. Die idealen Refraktionsdaten können auch als effektive objektive Refraktionsdaten bezeichnet werden. Anschließend ist es möglich, im vorgenannten Schritt S3 die Plausibilisierung der subjektiven Refraktionsdaten anhand der effektiven objektiven Refraktionsdaten, wie sie im Schritt S2' berechnet wurden, durchzuführen.

Alternativ ist es auch möglich, nach Berechnung der effektiven, objektiven Refraktionsdaten anhand der effektiven objektiven Refraktionsdaten im Schritt S4 Sollwerte zu bestimmen. Es ist auch möglich, daß im Schritt S4 die subjektiven Refraktionswerte und/oder die effektiven objektiven Refraktionswerte berücksichtigt werden.

Ferner können im Schritt S4 auch die weiteren subjektiven Daten des Benutzers bei der Bestimmung bzw. der Berechnung des Sollwertes berücksichtigt werden.

In anderen Worten wird anhand kombinierter Refraktionswerte, welche aufgrund der subjektiven Refraktionswerte, und/oder der weiteren subjektiven Daten und/oder der objektiven Refraktionsdaten, insbesondere der effektiven objektiven Refraktionsdaten bestimmt werden, ein Brillenglas oder beide Brillengläser des Benutzers bestimmt. Die Bestimmung kann eine Berechnung und/oder eine Optimierung und/oder eine Entnahme von Daten aus einer Datenbank umfassen.

Folglich kann zwar einerseits die subjektive Refraktionsbestimmung eine Reihe von Unzulänglichkeiten aufweisen und insbesondere kann es möglich sein, Brillengläser mit immer höherer Präzision zu bestimmen, insbesondere zu berechnen und zu optimieren. Um die technischen Möglichkeiten möglichst vollständig auszunutzen, ist es aber andererseits notwendig, Refraktionswerte bereitzustellen, welche die Sollwerte für das Brillenglas darstellen, wobei die Refraktionswerte möglichst exakt bestimmt werden müssen, da andernfalls beispielsweise Vorteile einer individuellen Optimierung in einer Gebrauchsstellung im wesentlichen neutralisiert werden. Erfindungsgemäß wurde erkannt, die Nachteile bzw. Ungenauigkeiten einer subjektiven Refraktionsbestimmung anhand der immer genaueren objektiven Meßmethoden auszugleichen, wobei sich mit immer besseren Meßmethoden, beispielsweise mit Hilfe von Aberrometern eine im wesentliche komplette Aberrations-Funktion beschreiben und auswerten läßt. Daraus ist es möglich, zum Beispiel für verschiedene Pupillendurchmesser oder bei verschiedenen Kontrasten eine ideale Refraktion zu berechnen. Hierbei kann vorteilhafterweise der Stiles-Crawford-Effekt, die Gewichtung der Pupille, usw. berücksichtigt werden. Weiterhin kann es möglich sein, die besten Sollwerte für das zu bestimmende Brillenglas im Hinblick auf verschiedene Helligkeiten und Pupillengrößen, verschiedenen Gewichtsfunktionen der Pupille, verschiedene Kontraste, zu optimalen Werten oder Verteilungen von RMS, PSF, OTF, MTF und/oder des Strehl Verhältnisses zu berechnen. Ebenso können andere Maße zur Beurteilung des Sehqualität herangezogen werden, wie etwa die Entropie der PSF, die Sehschärfe des visuellen Systems NS und einer Vielzahl weiterer, herkömmlicher Verfahren.

Erfindungsgemäß wurde daher vorteilhafterweise erkannt, die Vorteile einer subjektiven Refraktionsbestimmung, wie sie oben beschrieben wurde, und welche eine objektive Refraktionsbestimmung regelmäßig nicht leisten kann, mit der Genauigkeit einer objektiven Refraktionsbestimmung zu kombinieren. Hierbei werden beispielsweise von einem Augenoptiker bzw. einem Optometristen bzw. einem Augenarzt vorzugsweise nicht mehr nur die Werte der subjektiven Refraktionsbestimmung bereitgestellt, sondern auch Werte einer objektiven Messung. Die Werte der subjektiven und/oder der objektiven Messung können an einen Hersteller übermittelt werden bzw. zwischen dem Augenoptiker, dem Optometristen und/oder dem Augenarzt ausgetauscht werden.

Die objektive Messung kann beispielsweise anhand eines Skiaskops, anhand von exzentrischer Photorefraktion, anhand eines Autorefraktometers und/oder anhand eines Aberrometers durchgeführt werden. Die objektive Messung kann auch exzentrische Photorefraktionen umfassen. Vorzugsweise ist eine Messung mit großer Pupillenöffnung enthalten. Weiterhin vorzugsweise wird die Messung mit einem Aberrometer durchgeführt, da dadurch im wesentlichen die gesamte Aberrations-Funktion bekannt wird und sich daraus sehr leicht die obigen Optimierungen durchführen lassen, insbesondere berechnen lassen.

Weiterhin vorteilhafterweise kann, wie oben ausgeführt, eine Plausibilitätsprüfung der subjektiven Refraktionsdaten durchgeführt werden. Beispielsweise kann bei der subjektiven Refraktionsbestimmung eine Achse eines Zylinders um etwa 90° verdreht werden. Dies kann anhand der objektiven Refraktionsdaten leicht überprüft und gegebenenfalls korrigiert werden. Weiterhin kann beispielsweise überprüft werden, ob die Unterschiede zwischen der subjektiven Refraktionsmessung und der objektiven Refraktionsmessung plausibel sind. Ein etwas schwächerer Zylinder in der subjektiven Refraktionsmessung kann als akzeptabel angesehen werden und muß daher nicht korrigiert werden. Insbesondere ist herkömmlicherweise ein durch subjektive Refraktionsmessung bestimmter Zylinder oftmals schwächer als ein durch objektive Refraktionsmessung bestimmter Zylinder. Weiterhin muß nicht notwendigerweise korrigiert werden, daß auf beiden Seiten (d.h. für das linke und das rechte Auge) die sphärischen Werte, wie sie anhand der subjektiven Refraktionsmessung bestimmt wurden, etwa gleich in Richtung Plus oder Minus gegenüber den objektiven Werten abweichen. Gegebenenfalls kann auch, wie oben ausgeführt, korrigierend eingegriffen werden, um somit anhand beispielsweise eines geeigneten Algorithmus die Sollwerte für das Brillenglas zu verbessern. Der Eingriff kann manuell oder automatisch erfolgen.

Besonders vorteilhaft können anhand der Kombination der subjektiven und objektiven Refraktionsdaten, wie in Schritt S4 dargestellt, bessere Sollwerte für ein Brillenglas bestimmt, insbesondere berechnet werden, wie dies im Vergleich dazu lediglich anhand der individuellen subjektiven Refraktionsmessung oder der individuellen objektiven Refraktionsmessung nicht möglich ist. Beispielsweise ist es möglich, aus der objektiven Messung, insbesondere der exzentrischen Photorefraktion oder Aberrometermessung, eine für verschiedene Umgebungsparameter, welche beispielsweise Helligkeit, Kontrast, usw. umfassen, bessere Korrektur zu finden, als herkömmlicherweise möglich.

Vorzugsweise geht in einen möglichen Korrekturalgorithmus ein, ob eine Änderung durch Akkommodation ausgeglichen werden kann. Gehen beispielsweise die Refraktionswerte bei großer Pupille mehr in Richtung Minus, kann daraus gegebenenfalls abgeleitet werden, daß eine Mittelung der Werte zwischen kleiner und großer Pupille bei großen Pupillen zu einer Verbesserung führt, während es bei kleinen Pupillen, aufgrund des Ausgleichs durch die Akkommodation und gesteigerte Schärfentiefe, nicht zu einer Verschlechterung kommt. Bei einer Verschiebung in Richtung Plus hingegen kann dies kritischer sein.

Weiterhin vorzugsweise wird vermieden, daß durch eine Abänderung der Korrektionsstärke infolge der objektiven Meßergebnisse, beispielsweise bei größerer Pupille, die progressive Myopie zusätzlich gefördert wird. Hierbei können Größen erfaßt werden, die auch dazu genutzt werden können, abzuschätzen wie stabil bzw. progressiv die individuelle Myopie ist. Solche Größen können Alter, Phorie, Myopieprogression pro Jahr, Dauer, die seit Beginn der Myopie-Entwicklung vergangen ist, usw. sein, die in die subjektiven Daten aufgenommen werden. Vorzugsweise werden diese Größen berücksichtigt, um zu vermeiden, daß die Myopieprogression versehentlich mit der neu errechneten, gegebenenfalls stärkeren Minus-Korrektion gesteigert wird. Vorteilhafterweise wird daher nicht nur die bestmögliche Abbildung bei verschieden großen Pupillen erreicht, sondern es kann auch eine Korrektion erreicht werden, die, sofern notwendig, die Myopieprogression nicht stärker als die gewöhnliche Korrektion fördert oder weiterhin vorteilhafterweise die Myopieprogression hemmt.

Neben sphärischen und/oder zylindrischen Refraktionswerten können auch Aberrationen, insbesondere höherer Ordnung sowie die subjektive Refraktion berücksichtigt werden. Bei der Bewertung dieser Messung können verschiedene vormessene Pupillengrößen berücksichtigt werden, sowie die mittlere Pupillengröße und gegebenenfalls deren Varianz, die für die Altersgruppe des Benutzers typisch ist.

Weiterhin kann mittels Simulation unterschiedlicher Aberrationen und/oder einzelner bzw. kombinierter Aberrationsterme die Verträglichkeit und der Gewinn/Benefit möglicher Korrekturen ermittelt werden. Dies muß sowohl spontan als auch nach einiger Zeit der Adaption erfolgen. Hierbei kann berücksichtigt werden, daß nicht jede Aberration voll auskorrigiert werden soll, um gerade hohe Sehschärfen und/oder hohe Sehqualitäten zu ermöglichen. Weiterhin kann beachtet werden, daß insbesondere die Effekte von Aberrationen höherer Ordnung von der Varianz der Pupillengröße stark beeinträchtigt werden. Weiterhin kann bei der Entscheidung, welche Aberration wie stark auskorrigiert werden soll, die alle Aberrationen beeinflussende Schärfentiefe berücksichtigt werden.

Weiterhin vorzugsweise kann die Korrektur nicht ausschließlich nach Meßwerten erfolgen, sondern auch durch Gewichtung individueller, physiologisch begründeter weiterer bzw. verschiedener Faktoren. Folglich ergibt sich für die Korrektur die Möglichkeit, daß verschiedene Terme miteinander verrechnet werden können, um einige oder mehrere Aberrationen in Form weniger oder einiger Terme im Brillenglas bestmöglich korrigieren zu können. Alternativ oder zusätzlich kann die subjektive Refraktion bei verschiedenen Pupillengrößen ermittelt werden. Mögliche Unterschiede der subjektiven Refraktionswerte bei unterschiedlichen Pupillengrößen können optional oder zusätzlich bei der Optimierung der Korrektionswerte berücksichtigt werden. Weiterhin vorzugsweise wird zur Berechnung der Verordnungswerte und/oder Sollwerte eines Brillenglases aus der subjektiven und objektiven Refraktionsbestimmung die objektive Refraktionsbestimmung mit exzentrischer Photorefraktion durchgeführt, wobei gewählt wird, ob die Messung monokular oder binokular durchgeführt wird. Hierbei kann die Anzahl sowie die Ordnung der Aberrationen, die berücksichtigt werden, variabel sein.

Nachfolgend wird eine bevorzugte Variante der Kombination der subjektiven und objektiven Daten der Refraktionsbestimmung beschrieben, die es ermöglicht, eine bessere Verordnung bzw. bessere Sollwerte für ein Brillenglas zu bestimmen, insbesondere zu berechnen.

Mathematische Darstellung einer Kombination von objektiver Refraktion und subjektiver Refraktion

Parameter objektiver Refraktion:

| | |
|---|---|
| Sphärische Wirkung: | *sph_obj* |
| Zylindrische Wirkung: | *zyl_obj* |
| Achslage: | *A_obj* |

Parameter subjektiver Refraktion:

| | |
|---|---|
| Sphärische Wirkung: | *sph_sub* |
| Zylindrische Wirkung: | *zyl_sub* |
| Achslage: | *A_sub* |

Powervektor:

Als Powervektor wird definiert:

$$\mathbf{P} = \begin{pmatrix} M \\ J_0 \\ J_{45} \end{pmatrix}, \qquad\qquad (1)$$

wobei **P** lediglich eine verkürzende Schreibweise für eine Vielzahl möglicher Powervektoren für eine Vielzahl möglicher Datensätze ist. Zum Beispiel kann ein Powervektor $\mathbf{P}_{obj,r}$ Werte objektiver Refraktion für das rechte Auge beinhalten, insbesondere also $M_{r,obj}$, $J_{0,r,obj}$ und $J_{45,r,obj}$, ein Powervektor $\mathbf{P}_{obj,l}$ Werte objektiver Refraktion für das linke Auge beinhalten, insbesondere also $M_{l,obj}$, $J_{0,l,obj}$ und $J_{45,l,obj}$, ein Powervektor $\mathbf{P}_{sub,r}$ Werte subjektiver Refraktion für das rechte Auge beinhalten, insbesondere also $M_{r,sub}$, $J_{0,r,sub}$ und $J_{45,r,sub}$, ein Powervektor $\mathbf{P}_{sub,l}$ Werte subjektiver Refraktion für das linke Auge beinhalten, insbesondere also $M_{l,sub}$, $J_{0,l,sub}$ und $J_{45,l,sub}$. Weiterhin ist es möglich, daß in die Datensätze der objektiven Refraktionsdaten verschiedene Pupillendurchmesser eingehen, bei denen die objektiven Refraktionsdaten erstellt, insbesondere gemessen wurden. Folglich können *i* Powervektoren $\mathbf{P}_{obj,r}^{i}$ und/oder $\mathbf{P}_{obj,l}^{i}$ vorliegen, die objektive Refraktionswerte für *i* verschiedene Pupillendurchmesser $DP_{r}^{i}$ des rechten Auges bzw. für verschiedene Pupillendurchmesser $DP_{l}^{i}$ des linken Auges beinhalten, wobei *i* eine natürliche Zahl ist.

Analoge Ausführungen gelten auch für weitere Variablen bzw. Parameter, soweit anwendbar.

Die Vektorkomponenten des Powervektors sind nachfolgend definiert:

$$M = sph + \frac{zyl}{2}$$

$$J_0 = -\frac{zyl}{2}\cos 2A \ , \qquad\qquad (2)$$

$$J_{45} = -\frac{zyl}{2}\sin 2A$$

wobei die Vektorkomponenten zur verbesserten Übersichtlichkeit - wie oben - verkürzt dargestellt sind. Hinsichtlich der objektiven Refraktionsdaten des linken Auges bei einem Pupillendurchmesser $DP_l^i$ wird das sphärische Äquivalent beispielsweise als $M_{l,obj}$ bezeichnet.

Die obigen Darstellung kann folgendermaßen umformuliert werden:

$$zyl = -2\sqrt{J_0^2 + J_{45}^2}$$
$$sph = M - \frac{zyl}{2} \tag{3}$$
$$A = \frac{1}{2}\arctan\frac{J_{45}}{J_0}$$

$$A = \begin{cases} 45°; & J_0 = 0 \wedge J_{45} \leq 0 \\ 135°; & J_0 = 0 \wedge J_{45} > 0 \\ \frac{1}{2}\arctan\frac{J_{45}}{J_0}; & J_0 < 0 \wedge J_{45} \leq 0 \\ \frac{1}{2}\arctan\frac{J_{45}}{J_0} + 180°; & J_0 < 0 \wedge J_{45} > 0 \\ \frac{1}{2}\arctan\frac{J_{45}}{J_0} + 90°; & J_0 > 0 \end{cases}. \tag{4}$$

1. Plausibilisierung der subjektiven Refraktionsdaten aufgrund der objektiven Refraktionsdaten

Berechnung des Differenzvektors $\mathbf{P}_{Dif}$ zwischen den objektiven und subjektiven Refraktionsdaten jeweils für das rechte und linke Auge:

$$\mathbf{P}_{Dif,r} = abs(\mathbf{P}_{obj,r} - \mathbf{P}_{sub,r})$$
$$\mathbf{P}_{Dif,l} = abs(\mathbf{P}_{obj,l} - \mathbf{P}_{sub,l}) \tag{5}$$

Übersteigt der Maximalwert der einzelnen Vektorkomponenten der Vektoren $\mathbf{P}_{Dif,r}$ und $\mathbf{P}_{Dif,l}$ eine bestimmte Schranke S, kann beispielsweise ein Refraktionist (z.B. Augenoptiker, Augenarzt, Optometrist) informiert und/oder eine Überprüfung der subjektiven Refraktion veranlaßt werden.

In anderen Worten, wenn gilt:

$$Max\left(P_{Dif,r}(1), P_{Dif,r}(2), P_{Dif,r}(3), P_{Dif,l}(1), P_{Dif,l}(2), P_{Dif,l}(3)\right) \geq S \tag{6}$$

wird vorzugsweise ein Refraktionist informiert und/oder werden die subjektiven Refraktionsdaten überprüft.

Weiterhin vorzugsweise gilt:

$$S \leq 1,00dpt. \tag{7}$$

Alternativ oder zusätzlich kann bei Übersteigen der Norm der Differenz der Powervektoren $\mathbf{P}_{Dif,r}$ und $\mathbf{P}_{Dif,l}$ einer bestimmten Schranke S, der Refraktionist (z.B. Augenoptiker, Augenarzt, Optometrist) informiert und/oder eine Überprüfung der subjektiven Refraktion veranlaßt werden.

2. Simulation des binokularen Abgleichens

a. Berechnung der binokularen Differenz zwischen rechtem und linkem Auge

$$M_{Dif} = M_r - M_l,$$ 
(8)

wobei $M_{Dif}$ eine verkürzte Schreibweise für $M_{Dif,obj}$ oder $M_{Dif,sub}$ ist und $M_r$ eine verkürzte Schreibweise für $M_{r,obj}$ oder $M_{r,sub}$ und $M_l$ eine verkürzte Schreibweise für $M_{l,obj}$ oder $M_{l,sub}$ ist.

b. Berechnung der Differenz zwischen der subjektiven und objektiven Differenz

$$Dif = M_{Dif,obj} - (g_{obj\_bino} \; M_{Dif,obj} + g_{sub\_bino} \; M_{Dif,subj})$$
$$g_{obj\_bino} = 1 - g_{sub\_bino}$$
$$0 < g_{sub\_bino} < 1$$
(9)

Bevorzugt gilt:

$$g_{sub\_bino} \geq 0{,}6$$
(10)

Besonders bevorzugt gilt:

$$g_{sub\_bino} = 0{,}9\,.$$
(11)

c. Anpassung der objektiven Daten

Es gilt:

$$M^*_{r,obj} := M_{r,obj} - \frac{Dif}{2}$$
$$M^*_{l,obj} := M_{l,obj} + \frac{Dif}{2}$$
(12)

Zur besseren Übersichtlichkeit wird $M^*_{r,obj}$ im nachfolgenden Formalismus wieder als $M_{r,obj}$ bezeichnet. Ebenso wird $M^*_{l,obj}$ im nachfolgenden Formalismus wieder als $M_{l,obj}$ bezeichnet.
Daraus läßt sich dann die sphärische Wirkung rechts und links mit

$$sph = M - \frac{zyl}{2}$$
(13)

berechnen, wobei vorzugsweise Zylinder und Achslage unverändert bleiben. Wie bereits oben ausgeführt wurde, kann für das rechte Auge die sphärische Wirkung $sph_r$ und für das linke Auge die sphärische Wirkung $sph_l$ bestimmt werden. Insbesondere kann die sphärische Wirkung folgendermaßen definiert sein:

$$sph_r = M_{r,obj} - \frac{zyl_r}{2}$$
(14)

und/oder

$$sph_l = M_{l,obj} - \frac{zyl_l}{2}.$$  (15)

Es ist aber auch möglich, daß die nachfolgende Kombination der subjektiven und der objektiven Refraktion ohne binokularen Abgleich durchgeführt wird.

3. Kombination der subjektiven und objektiven Refraktion

Die resultierende neue Refraktion $P_K$ berechnet sich aus der subjektiven und objektiven Refraktion wie folgt:

$$\mathbf{P}_K = \mathbf{G}_{obj}\mathbf{P}_{obj} + \mathbf{G}_{sub}\mathbf{P}_{sub}$$  (16)

wobei gilt:

$$\mathbf{G} = \begin{pmatrix} g_1 & 0 & 0 \\ 0 & g_2 & 0 \\ 0 & 0 & g_3 \end{pmatrix},$$  (17)

$$0 < g_i < 1,$$  (18)

und

$$\mathbf{G}_{obj} + \mathbf{G}_{sub} = 1.$$  (19)

Bevorzugt gilt:

$$g_{sub,i} \geq 0{,}5.$$  (20)

Besonders bevorzugt gilt:

$$g_{sub,1} \geq 0{,}6 \quad g_{sub,2} \geq 0{,}5 \quad g_{sub,3} \geq 0{,}7 \text{ und } Abs(g_{sub,2} - g_{sub,3}) \leq 0{,}3.$$  (21)

Ganz besonders bevorzugt gilt:

$$g_{sub,1} = 0{,}7 \quad g_{sub,2} = 0{,}6 \quad g_{sub,3} = 0{,}8 \quad Abs(g_{sub,2} - g_{sub,3}) \leq 0{,}1.$$  (22)

4. Bestimmung der Addition

Addition $Add$

$$Add_r = Add - M_{K,r} + M_{sub,r}$$
$$Add_l = Add - M_{K,l} + M_{sub,l}.$$  (23)
$$Add_K = \frac{Add_r + Add_l}{2}$$

Vorteilhafterweise wird somit eine Verschiebung in der Ferne ausgeglichen.

Um den bei der subjektiven Refraktionsbestimmung bestimmten Nahwert nicht zu verändern, muss die Addition entsprechend um den Betrag angepasst werden, um den das sphärische Äquivalent *M* in der Ferne geändert wird, da die Addition die Differenz zwischen Fern- und Nahwert darstellt.

### 5. Bestimmung einer Brille bei Vorliegen von binokularen Problemen

Liegen weitere Informationen über den Binokularstatus vor, kann durch ein abweichendes bzw. ergänzendes Vorgehen zu Punkt 3, wie oben beschrieben, die Bestimmung des sphärischen Äquivalentes *M* und der Addition *Add* auch auf anderem Weg durchgeführt werden:

### a. Konvergenzinsuffizienz

Merkmale: Geringer ACA-Gradient, kein Akkommodationsmangel in der Nähe, Exophorie, d.h., eine Horizontalphorie, bei der die fusionsreizfreie Ruhestellung von der Orthostellung nach außen abweicht, in der Nähe, geringe maximale Konvergenz, schlechte positive und gute negative relative Vergenz in der Nähe, schlechte negative und gute positive relative Akkommodation in der Nähe. Der Begriff ACA-Gradient beschreibt diejenige akkommodative Vergenz, die mit einer Änderung des Akkommodationszustandes um eine Dioptrie gekoppelt ist. Die Abkürzung ACA steht für den englischen Begriff *accommodative vergence* (akkommodative Vergenz) geteilt durch *accommodation* (Akkommodation).

Abweichend von dem unter Punkt 3 oben beschriebenen Vorgehen können das sphärische Äquivalent $M_K$ der Kombination und die Addition folgendermaßen bestimmt werden:

$$g_{subj,2} \geq 0,6 \quad g_{subj,3} \geq 0,8 \tag{24}$$

$$M_K = \min(\frac{M_{obj} + M_{sub}}{2}, M_{sub}) \tag{25}$$

$$Add_r = Add - \max(0, M_{K,r} - M_{sub,r})$$
$$Add_l = Add - \max(0, M_{K,l} - M_{sub,l}) \ . \tag{26}$$
$$Add_K = \min(Add_r, Add_l)$$

### b. Konvergenzexzess

Merkmale:

Hoher ACA-Gradient, Akkommodationsmangel in der Nähe, Esophorie, d.h. eine Horizontalphorie, bei der die fusionsreizfreie Ruhestelleung von der Orthostellung nach innen abweicht, in der Nähe, große maximale Konvergenz, gute positive und schlechte negative relative Vergenz in der Nähe, gute negative und schlechte positive relative Akkommodation in der Nähe.

Abweichend von dem unter Punkt 3 oben beschriebenen Vorgehen kann das sphärische Äquivalent $M_K$ der Kombination folgendermaßen bestimmt werden:

$$g_{subj,2} \geq 0,6 \quad g_{subj,3} \geq 0,8 \tag{27}$$

$$M_K = \max(\frac{M_{obj} + M_{sub}}{2}, M_{sub}) \tag{28}$$

$$Add_r = Add - \min(0, M_{K,r} - M_{sub,r})$$
$$Add_l = Add - \min(0, M_{K,l} - M_{sub,l}) \ . \qquad (29)$$
$$Add_K = \max(Add_r, Add_l)$$

c. Exophorie

Merkmale:

Exophorie in der Ferne und Nähe, Normaler ACA-Gradient, geringe maximale Konvergenz, schlechte positive und gute negative relative Vergenz, schlechte negative und gute positive relative Akkommodation.
Abweichend von dem unter Punkt 3 oben beschriebenen Vorgehen kann das sphärische Äquivalent $M_K$ der Kombination folgendermaßen bestimmt werden:

$$M_K = \min(M_{obj}, M_{sub}) \qquad (30)$$

$$g_{subj,2} \geq 0{,}6 \quad g_{subj,3} \geq 0{,}8 \ . \qquad (31)$$

d. Esophorie

Merkmale:

Esophorie in der Ferne und Nähe, Normaler ACA-Gradient, große maximale Konvergenz, gute positive und schlechte negative relative Vergenz, gute negative und schlechte positive relative Akkommodation.
Abweichend von dem unter Punkt 3 oben beschriebenen Vorgehen kann das sphärische Äquivalent $M_K$ der Kombination folgendermaßen bestimmt werden.

$$M_K = \max(M_{obj}, M_{sub}) \qquad (32)$$

$$g_{subj,2} \geq 0{,}6 \quad g_{subj,3} \geq 0{,}8 \ . \qquad (33)$$

6. Bestimmung einer Brille für das Dämmerungssehen (Mesopisches Sehen)

Zusätzlich oder alternativ zu dem oben unter Punkt 2 und 3 beschriebenen Verfahren, kann jeweils für das rechte und linke Auge wir folgt vorgegangen werden:

a. Berechnung der objektiven Refraktionsdaten aus den Wellenfrontdaten der Aberrometermessung mit einem kleinen Pupillendurchmesser für das photopische Sehen:

$$\mathbf{P}_{obj,ph} \ . \qquad (34)$$

b. Berechnung der objektiven Refraktionsdaten aus den Wellenfrontdaten der Aberrometermessung mit einem großen Pupillendurchmesser für das mesopische Sehen:

$$\mathbf{P}_{obj,sk} \ . \qquad (35)$$

c. Berechnung der Differenz zwischen der photopischen und mesopischen objektiven Refraktion:

$$\mathbf{P}_{Dif} = \mathbf{P}_{obj,sk} - \mathbf{P}_{obj,ph} \ . \qquad (36)$$

d. Berechnung einer subjektiven Refraktion für das mesopische Sehen aus der normalen subjektiven Refraktion und der Differenz zwischen der photopischen und mesopischen objektiven Refraktion:

$$\mathbf{P}_{sub,sk} = \mathbf{P}_{sub} + \mathbf{P}_{Dif} \, . \tag{37}$$

Anschließend wird, wie oben unter Punkt 2 und 3 beschrieben wurde, die Kombination der Refraktion $\mathbf{P}_{K,sk}$ für das mesopische Sehen aus der subjektiven Refraktion für das mesopische Sehen $\mathbf{P}_{sub,sk}$ und der objektiven Refraktion $\mathbf{P}_{obj,sk}$ berechnet.

Dieses Verfahren kann für zumindest einen weiteren (dritten) Pupillendurchmesser wiederholt werden und so zumindest eine weitere Kombination $\mathbf{P}_{K,sk,2}$ berechnet werden.

Aus den 3 (mindestens 2) vorliegenden Refraktionen $\mathbf{P}_K$, $\mathbf{P}_{K,sk}$ und $\mathbf{P}_{K,sk,2}$ für verschiedene Pupillendurchmesser kann durch geeignete Interplolation (z.B. linear) in Abhängigkeit vom Pupillendurchmesser bzw. der Beleuchtungsstärke die ideale Refraktion berechnet werden.

## 7. Ausführungsbeispiel

Im folgenden Ausführungsbeispiel ist ein Augenpaar dargestellt, wobei subjektive Refraktionsdaten für das linke und das rechte Auge vorlagen sowie objektive Refraktionsdaten für das linke und das rechte Auge für drei verschiedene Pupillendurchmesser. Die beispielhaften Werte sind in Tabelle 1 enthalten, wobei die objektiven Daten aus einer Aberrometermessung stammen. In Tabelle 1 sind ebenfalls die resultierenden Refraktionswerte für die verschiedenen beschriebenen Methoden angegeben. Die "Standard"-Werte entstehen (entsprechend der Schritte 2 und 3) durch Kombination der subjektiven Refraktionsdaten mit den objektiven Refraktionsdaten bei einem Pupillendurchmesser

$$DP_r^1 = DP_l^1 = 3mm \, , \tag{38}$$

wobei zur Kombination die Gleichung

$$\mathbf{P}_K = \mathbf{G}_{obj}\mathbf{P}_{obj} + \mathbf{G}_{sub}\mathbf{P}_{sub} \tag{39}$$

mit den beispielhaften Gewichten

$$g_{sub,1} = 0,7 \quad g_{sub,2} = 0,6 \quad g_{sub,3} = 0,8 \tag{40}$$

benutzt wird. Die mit "Leicht" und "Dunkel" bezeichneten Refraktionsdaten zur Dämmerung entstehen dadurch, die objektiven Refraktionsdaten zu den Pupillendurchmessern

$$DP_r^2 = DP_l^2 = 5mm \tag{41}$$

und

$$DP_r^3 = DP_l^3 = 5,55mm \tag{42}$$

mit den subjektiven Daten so zu kombinieren, wie oben in Abschnitt 6 beschrieben wurde.

Die mit "Konvergenzinsuffizienz", "Konvergenzexzess", "Exophorie" und "Esophorie" bezeichneten Refraktionsdaten entstehen, wie oben in Abschnitt 5 beschrieben wurde.

Tabelle 1:

| | rechts | | | links | | | |
| --- | --- | --- | --- | --- | --- | --- | --- |
| | Sph [dpt] | Zyl [dpt] | A [°] | Sph [dpt] | Zyl [dpt] | A [°] | Add [dpt] |
| **Subjektiv** | -5.25 | -1.00 | 3 | -4.25 | -1.75 | 165 | "2.00" |
| **Objektiv Pupillendurchmesser 3 mm** | -5.02 | -1.58 | 6 | -5.47 | -3.07 | 170 | |
| **Objektiv Pupillendurchmesser 5 mm** | -4.92 | -1.60 | 3 | -5.12 | -3.75 | 178 | |
| **Objektiv Pupillendurchmesser 5.55 mm** | -4.88 | -1.61 | 2 | -4.89 | -3.90 | 1 | |
| | | | | | | | |
| **Ergebnis** | | | | | | | |
| **Standard** | **-5.40** | **-1.22** | **3** | **-4.31** | **-2.25** | **168** | 2.21 |
| **Dämmerung** | | | | | | | |
| **Leicht** | -5.30 | -1.26 | 1 | -3.97 | -2.93 | 179 | 2.24 |
| **Dunkel** | -5.25 | -1.27 | 179 | -3.71 | -3.13 | 7 | 2.15 |
| | | | | | | | |
| **Sonderfälle** | | | | | | | |
| **Konvergenzinsuffizienz** | -5.58 | -1.22 | 3 | -4.53 | -2.25 | 168 | 2.00 |
| **Konvergenzexzess** | -5.14 | -1.22 | 3 | -4.00 | -2.25 | 168 | 2.00 |
| **Exophorie** | -6.02 | -1.22 | 3 | -5.06 | -2.25 | 168 | 2.00 |
| **Esophorie** | -5.14 | -1.22 | 3 | -4.00 | -2.25 | 168 | 2.00 |

Die in Tabelle 1 angegebenen objektiven Daten stammen aus Aberrometermessungen, die auch Zernike-Koeffizienten zu höheren Ordnungen (Koma, Trefoil, sphärische Aberration, ...) enthalten. Allgemein hängen die zu wählenden objektiven Refraktionsdaten auch von diesen Koeffizienten höherer Ordnung ab. Die Art dieser Abhängigkeit wird durch die Metrik bestimmt. Wird als Metrik die RMS (root mean square) der Wellenfrontaberration benutzt, die für optimales Sehen minimiert werden muß, so wird diese genau dadurch minimiert, daß die Koeffizienten der niedrigen Ordnung (Sphäre, Zylinder, Achse) angepaßt werden. Somit müssen die Koeffizienten höherer Ordnung in Tabelle 1 nicht notwendigerweise in die Bestimmung eingehen.

Ein Beispiel für eine Metrik, bei der die Koeffizienten höherer Ordnung benötigt werden, ist das Maximum MaxPSF der PSF (Point Spread Function), welches für optimales Sehen maximiert werden muß. In Tabelle 2 sind die Zernike-Koeffizienten höherer Ordnung für das rechte Auge bei zwei Pupillendurchmessern (den oben beispielhaft definierten Pupillendurchmessern $DP_r^2 = 5mm$ und $DP_r^3 = 5{,}55mm$) und für das linke Auge bei einem Pupillendurchmesser (dem oben beispielhaft definierten Pupillendurchmesser $DP_l^3 = 5{,}55mm$) angegeben.

Tabelle 2:

| Symbol | Name | Rechtes Auge, Pupille 5.55mm | Rechtes Auge, Pupille 5.0mm | Linkes Auge, Pupille 5.55mm |
| --- | --- | --- | --- | --- |
| $C_{3,1}[\mu m]$ | Koma | 0.112933 | 0.0597886 | -1.04653 |
| $C_{3,-1}[\mu m]$ | Koma | -0.375738 | -0.256975 | 0.344224 |
| $C_{3,3}[\mu m]$ | Trefoil | 0.0140263 | 0.030974 | -0.0234007 |
| $C_{3,-3}[\mu m]$ | Trefoil | -0.169401 | -0.122997 | -0.0124213 |
| $C_{4,0}[\mu m]$ | Sph. Aberration | -0.00786817 | -0.0130847 | -0.0616062 |

(fortgesetzt)

| Symbol | Name | Rechtes Auge, Pupille 5.55mm | Rechtes Auge, Pupille 5.0mm | Linkes Auge, Pupille 5.55mm |
|---|---|---|---|---|
| $C_{4,2}[\mu m]$ | Sek. Astigm | 0.096995 | 0.0671711 | -0.181049 |
| $C_{4,-2}[\mu m]$ | Sek. Astigm | -0.0780137 | -0.0539008 | -0.209747 |
| $C_{4,4}[\mu m]$ | Quadrafoil | -0.0390678 | -0.0249956 | 0.10042 |
| $C_{4,-4}[\mu m]$ | Quadrafoil | 0.0750261 | 0.061365 | 0.059569 |

In Tabelle 3 sind nun die der Tabelle 1 entsprechenden Resultate angegeben, nur daß anstatt von aus der RMS bestimmten objektiven Refraktionsdaten nun Refraktionsdaten stehen, die durch Maximierung der Metrik MaxPSF entstehen. In der mit "Ergebnis" bezeichneten Zeile wurden beispielhaft die kombinierten Refraktionsdaten aus den subjektiven Daten mit den objektiven Daten zum Pupillendurchmesser $DP_r^3 = 5,55mm$ benutzt. Die mit "Konvergenzinsuffizienz", "Konvergenzexzess", "Exophorie" und "Esophorie" bezeichneten Refraktionsdaten entstehen, wie oben in Abschnitt 5 beschrieben wurde.

Tabelle 3:

| | rechts | | | links | | | |
|---|---|---|---|---|---|---|---|
| | Sph [dpt] | Zyl [dpt] | A [°] | Sph [dpt] | Zyl [dpt] | A [°] | Add [dpt] |
| **Subjektiv** | -5.25 | -1.00 | 3 | -4.25 | -1.75 | 165 | "2.00" |
| **Objektiv mit Metrik Pupillendurchmesser 5 mm** | -4.72 | -1.39 | 0 | | | | |
| **Objektiv mit Metrik Pupillendurchmesser 5.55 mm** | -4.68 | -1.41 | 3 | -5.09 | -3.90 | 4 | |
| | | | | | | | |
| **Ergebnis** | **-5.37** | **-1.16** | **3** | **-4.13** | **-2.52** | **7** | 2.23 |
| | | | | | | | |
| **Sonderfälle** | | | | | | | |
| **Konvergenzinsuffizienz** | -5.50 | -1.16 | 3 | -4.31 | -2.52 | 7 | 2.00 |
| **Konvergenzexzess** | -5.17 | -1.16 | 3 | -3.86 | -2.52 | 7 | 2.00 |
| **Exophorie** | -5.83 | -1.16 | 3 | -4.75 | -2.52 | 7 | 2.00 |
| **Esophorie** | -5.17 | -1.16 | 3 | -3.86 | -2.52 | 7 | 2.00 |

Die vorliegende Erfindung ist nicht auf die vorgenannten, bevorzugten Ausführungsvarianten bzw. Formen beschränkt. Vielmehr können einzelne Merkmale bzw. Gruppen von Merkmalen der individuellen Ausführungsvarianten bzw. Ausführungsformen beliebig miteinander kombiniert werden und weitere Ausführungsformen bzw. Ausführungsvarianten der Erfindung dadurch geschaffen werden.

**Bezugszeichenliste**

S1    Schritt

S2    Schritt

S2'    Schritt

S3    Schritt

S4    Schritt


**Patentansprüche**

1.    Verfahren zum Herstellen eines Brillenglases mit den Schritten:

- Bereitstellen (S1) subjektiver Daten eines Brillenglasbenutzers, wobei die subjektiven Daten zumindest subjektive Refraktionsdaten $\mathbf{P}_{sub}$ umfassen;
- Bereitstellen (S2) objektiver Refraktionsdaten $\mathbf{P}_{obj}$ des Brillenglasbenutzers;
- Vergleichen (S3) zumindest einer Teilmenge der subjektiven Refraktionsdaten mit zumindest einer Teilmenge der objektiven Refraktionsdaten und Bestimmen eines Vergleichsergebnisses;
- Anpassen zumindest der Teilmenge der subjektiven Refraktionsdaten an die objektiven Refraktionsdaten aufgrund des Vergleichsergebnisses unter der Voraussetzung, daß das Vergleichsergebnis zumindest eine vorbestimmte Vergleichsbedingung erfüllt, ansonsten Beibehalten zumindest der Teilmenge der subjektiven Refraktionsdaten, wobei die Vergleichsbedingung darin besteht, dass zwei zu vergleichende Parameter aus den Refraktionsdaten eine bestimmte Differenz aufweisen oder überschreiten, wobei das Anpassen der Teilmenge der subjektiven Refraktionsdaten an die objektiven Refraktionsdaten ein Berechnen von kombinierten Refraktionsdaten $\mathbf{P}_K$ umfasst, und wobei die kombinierten Refraktionsdaten $\mathbf{P}_K$ durch Addition der objektiven Refraktionsdaten $\mathbf{P}_{obj}$ mit den subjektiven Refraktionsdaten $\mathbf{P}_{sub}$ folgendermaßen berechnet werden:

$$\mathbf{P}_K = \mathbf{G}_{obj}\mathbf{P}_{obj} + \mathbf{G}_{sub}\mathbf{P}_{sub}$$

wobei gilt:

$$\mathbf{G} = \begin{pmatrix} g_1 & 0 & 0 \\ 0 & g_2 & 0 \\ 0 & 0 & g_3 \end{pmatrix},$$

$$0 < g_i < 1,$$

$$\mathbf{G}_{obj} + \mathbf{G}_{sub} = 1$$

und wobei:

$$\mathbf{P} = \begin{pmatrix} M \\ J_0 \\ J_{45} \end{pmatrix}$$

und

$$M = sph + \frac{zyl}{2}$$

$$J_0 = -\frac{zyl}{2}\cos 2A$$

$$J_{45} = -\frac{zyl}{2}\sin 2A \; ;$$

- Bestimmen (S4) von Sollwerten zumindest eines Brillenglases, wobei die Sollwerte abhängig vom Vergleichsergebnis entweder anhand der beibehaltenen Teilmenge der subjektiven Refraktionsdaten oder anhand der kombinierten Refraktionsdaten bestimmt werden;
- Herstellen des zumindest einen Brillenglases anhand der bestimmten Sollwerte.

2. Verfahren nach Anspruch 1, mit dem weiteren Schritt:

- Bereitstellen einer Nachricht, welche das Vergleichsergebnis enthält.

3. Verfahren nach Anspruch 1 oder 2, wobei mehrere Sätze objektiver Refraktionsdaten bereitgestellt werden und effektive objektive Refraktionsdaten anhand der mehreren Sätze objektiver Refraktionsdaten berechnet werden, und wobei zum Bestimmen des Vergleichsergebnisses die effektiven objektiven Refraktionsdaten mit zumindest der Teilmenge der subjektiven Refraktionsdaten verglichen werden.

4. Verfahren nach Anspruch 3, wobei die effektiven objektiven Refraktionsdaten unter Beachtung folgender Randbedingungen bestimmt werden:

- Helligkeit und/oder
- Pupillendurchmesser und/oder
- Kontrast und/oder
- Alter.

5. Verfahren nach einem der Ansprüche 3 und 4, wobei zur Berechnung der effektiven objektiven Refraktionsdaten eine Aberrationsfunktion, insbesondere mit variabler Ordnung, und/oder eine OTF und/oder eine MTF und/oder eine PSF und/oder die Standardabweichung verwendet wird/werden.

6. Verfahren nach einem der vorangegangenen Ansprüche, wobei zumindest eine Teilmenge der objektiven Refraktionsdaten anhand exzentrischer Photorefraktion bestimmt wird.

7. Verfahren nach einem der vorangegangenen Ansprüche, wobei gilt:
$g_{sub,i} \geq 0{,}5$, wobei insbesondere gilt $g_{sub,1} \geq 0{,}6$ $g_{sub,2} \geq 0{,}5$ $g_{sub,3} \geq 0{,}7$ und $Abs(g_{sub,2} - g_{sub,3}) \leq 0{,}3$, wobei weiterhin insbesondere gilt:

$$g_{sub,1} = 0{,}7 \quad g_{sub,2} = 0{,}6 \quad g_{sub,3} = 0{,}8 \quad Abs(g_{sub,2} - g_{sub,3}) \leq 0{,}1 \, .$$

8. Verfahren nach einem der vorangegangenen Ansprüche, wobei ein sphärisches Äquivalent $M$ der objektiven Refraktionsdaten binokular bestimmt wird, wobei gilt:

$$M^{*}{}_{r,obj} := M_{r,obj} - \frac{Dif}{2}$$

$$M^{*}{}_{l,obj} := M_{l,obj} + \frac{Dif}{2} \, ,$$

wobei

$$M_{Dif} = M_r - M_l$$

$$Dif = M_{Dif,obj} - (g_{obj\_bino} M_{Dif,obj} + g_{sub\_bino} M_{Dif,subj})$$

$$g_{obj\_bino} = 1 - g_{sub\_bino}$$

$$0 < g_{sub\_bino} < 1$$

9. Verfahren nach Anspruch 8, wobei gilt:
$g_{sub\_bino} \geq 0{,}6$, wobei insbesondere gilt:

$$g_{sub\_bino} = 0{,}9 \,.$$

10. Computerprogrammprodukt, welches, wenn geladen in den Speicher eines Computers und ausgeführt von einem Computer, bewirkt, daß der Computer ein Verfahren nach einem der vorangegangenen Ansprüche mit Ausnahme des letzten Verfahrensschritts von Anspruch 1 durchführt.

11. Vorrichtung zum Herstellen eines Brillenglases mit:

- einer Subjektivdaten-Bereitstellungseinrichtung, welche ausgelegt ist, subjektive Daten eines Brillenglasbenutzers bereitzustellen, wobei die subjektiven Daten zumindest subjektive Refraktionsdaten $\mathbf{P}_{sub}$ umfassen;
- einer Objektivdaten-Bereitstellungseinrichtung, welche ausgelegt ist, objektive Refraktionsdaten $\mathbf{P}_{obj}$ des Brillenglasbenutzers bereitzustellen;
- einer Vergleichseinrichtung, welche ausgelegt ist, zumindest eine Teilmenge der subjektiven Refraktionsdaten mit zumindest einer Teilmenge der objektiven Refraktionsdaten zu vergleichen und welche ausgelegt ist, ein Vergleichsergebnis zu bestimmen;
- einer Anpassungseinrichtung, welche ausgelegt ist zumindest die Teilmenge der subjektiven Refraktionsdaten an die objektiven Refraktionsdaten aufgrund des Vergleichsergebnisses unter der Voraussetzung anzupassen, daß das Vergleichsergebnis zumindest eine vorbestimmte Vergleichsbedingung erfüllt, und wobei die Anpassungseinrichtung ausgelegt ist, ansonsten zumindest die Teilmenge der subjektiven Refraktionsdaten beizubehalten, wobei die Vergleichsbedingung darin besteht, dass zwei zu vergleichende Parameter aus den Refraktionsdaten eine bestimmte Differenz aufweisen oder überschreiten;
wobei, das Anpassen der Teilmenge der subjektiven Refraktionsdaten an die objektiven Refraktionsdaten ein Berechnen von kombinierten Refraktionsdaten $\mathbf{P}_K$ umfasst, und wobei die kombinierten Refraktionsdaten $\mathbf{P}_K$ durch Addition der objektiven Refraktionsdaten $\mathbf{P}_{obj}$ mit den subjektiven Refraktionsdaten $\mathbf{P}_{sub}$ folgendermaßen berechnet werden:

$$\mathbf{P}_K = \mathbf{G}_{obj}\mathbf{P}_{obj} + \mathbf{G}_{sub}\mathbf{P}_{sub}$$

wobei gilt:

$$\mathbf{G} = \begin{pmatrix} g_1 & 0 & 0 \\ 0 & g_2 & 0 \\ 0 & 0 & g_3 \end{pmatrix},$$

$$0 < g_i < 1 \,,$$

$$\mathbf{G}_{obj} + \mathbf{G}_{sub} = 1$$

und wobei:

$$\mathbf{P} = \begin{pmatrix} M \\ J_0 \\ J_{45} \end{pmatrix}$$

und

$$M = sph + \frac{zyl}{2}$$

$$J_0 = -\frac{zyl}{2}\cos 2A$$

$$J_{45} = -\frac{zyl}{2}\sin 2A \quad ;$$

wobei die Vorrichtung ferner ausgelegt ist, Sollwerte zumindest eines Brillenglases abhängig vom Vergleichsergebnis entweder anhand der beibehaltenen Teilmenge der subjektiven Refraktionsdaten oder anhand der kombinierten Refraktionsdaten zu bestimmen;

- einer Herstellungseinrichtung, welche ausgelegt ist, zumindest ein Brillenglas anhand der bestimmten Sollwerte herzustellen.

**Claims**

1.  A method for producing a spectacle lens comprising the steps of:

    - providing (S1) subjective data of a spectacle lens user, the subjective data comprising at least subjective refraction data $P_{sub}$;
    - providing (S2) objective refraction data $P_{obj}$ of the spectacle lens user;
    - comparing (S3) at least one subset of the subjective refraction data with at least one subset of the objective refraction data and determining a comparison result;
    - matching at least the subset of the subjective refraction data to the objective refraction data based on the comparison result, provided that the comparison result satisfies at least one predetermined comparison condition, otherwise maintaining at least the subset of the subjective refraction data, the comparison condition being that two parameters to be compared are selected from the refraction data have or exceed a certain difference, wherein the adaptation of the subset of the subjective refraction data to the objective refraction data comprises calculating combined refraction data $P_K$, and wherein the combined refraction data $P_K$ are calculated by adding the objective refraction data $P_{obj}$ with the subjective refraction data $P_{sub}$ as follows:

    $$P_K = G_{obj}\, P_{obj} + G_{sub}\, P_{sub}$$

    where:

    $$\mathbf{G} = \begin{pmatrix} g_1 & 0 & 0 \\ 0 & g_2 & 0 \\ 0 & 0 & g_3 \end{pmatrix},$$

$$0 < g_i < 1,$$

$$G_{obj} + G_{sub} = 1$$

and wherein:

$$P = \begin{pmatrix} M \\ J_0 \\ J_{45} \end{pmatrix}$$

and

$$M = sph + \frac{zyl}{2}$$

$$J_0 = -\frac{zyl}{2}\cos 2A$$

$$J_{45} = -\frac{zyl}{2}\sin 2A \quad ;$$

- determining (S4) target values of at least one spectacle lens, the target values being determined on the basis of the comparison result either on the basis of the retained subset of the subjective refraction data or on the basis of the combined refraction data;
- producing the at least one spectacle lens based on the specific target values.

2. The method of claim 1 with the further step:

- providing a message containing the comparison result.

3. The method of claim 1 or 2, wherein a plurality of sets of objective refraction data are provided and effective objective refraction data are calculated from the plurality of sets of objective refraction data, and wherein for determining the comparison result, the effective objective refraction data is compared with at least the subset of the subjective refraction data.

4. The method according to claim 3, wherein the effective objective refraction data are determined under consideration of the following boundary conditions:

- brightness and / or
- pupil diameter and / or
- contrast and / or
- age.

5. The method according to any one of claims 3 and 4, wherein an aberration function, in particular variable order, and / or an OTF and / or an MTF and / or a PSF and / or the standard deviation is / are used to calculate the effective objective refraction data.

6. The method according to any one of the preceding claims, wherein at least one subset of the objective refraction data is determined by means of eccentric photorefraction.

7. The method according to any one of the preceding claims, wherein

$g_{sub,i} \geq 0,5$, wherein in particular
$g_{sub,1} \geq 0,6$ $g_{sub,2} \geq 0,5$ $g_{sub,3} \geq 0,7$ and $Abs(g_{sub,2} - g_{sub,3}) \leq 0,3$, wherein furthermore in particular:
$g_{sub,1} = 0,7$ $g_{sub,2} = 0,6$ $g_{sub,3} = 0,8$ $Abs(g_{sub,2} - g_{sub,3}) \leq 0,1$.

8. The method according to any one of the preceding claims, wherein a spherical equivalent M of the objective refraction data is determined binocular, wherein:

$$M^{\bullet}_{r,obj} := M_{r,obj} - \frac{Dif}{2}$$

$$M^{\bullet}_{l,obj} := M_{l,obj} + \frac{Dif}{2}\text{,}$$

in which

$$M_{Dif} = M_r - M_l$$

$$Dif = M_{Dif,obj} - (g_{obj\_bino}\, M_{Dif,obj} + g_{sub\_bino}\, M_{Dif,subj})$$

$$g_{obj\_bino} = 1 - g_{sub\_bino}$$

$$0 < g_{sub\_bino} < 1$$

9. The method according to claim 8, wherein:
$g_{sub\_bino} \geq 0,6$, wherein in particular:

$$g_{sub\_bino} = 0,9.$$

10. A computer program product which, when loaded into the memory of a computer and executed by a computer, causes the computer to perform a method according to any one of the preceding claims, except for the last method step of claim 1.

11. Device for producing a spectacle lens with:

- a subjective data providing device, which is designed to provide subjective data of a spectacle lens user, the subjective data comprising at least subjective refraction data $P_{sub}$;
- an objective data providing device, which is designed to provide objective refraction data $P_{obj}$ of the spectacle lens user;
- a comparison device, which is designed to compare at least one subset of the subjective refraction data with at least one subset of the objective refraction data and which is designed to determine a comparison result;
- an adaptation device, which is adapted to adapt at least the subset of the subjective refraction data to the objective refraction data based on the comparison result, provided that the comparison result satisfies at least a predetermined comparison condition, and wherein the adaptation device is adapted otherwise to maintain at least the subset of the subjective refraction data; wherein the comparison condition is that two parameters to be compared from the refraction data have or exceed a certain difference;

wherein, adjusting the subset of the subjective refraction data to the objective refraction data comprises calculating combined refraction data $P_K$, and wherein the combined refraction data $P_K$ is calculated by adding the objective refraction data $P_{obj}$ to the subjective refraction data $P_{sub}$ as follows:

$$P_K = G_{obj}\, P_{obj} + G_{sub}\, P_{sub}$$

wherein:

$$G = \begin{pmatrix} g_1 & 0 & 0 \\ 0 & g_2 & 0 \\ 0 & 0 & g_3 \end{pmatrix},$$

$$0 < g_i < 1,$$

$$G_{obj} + G_{sub} = 1$$

and wherein:

$$P = \begin{pmatrix} M \\ J_0 \\ J_{45} \end{pmatrix}$$

and

$$M = sph + \frac{zyl}{2}$$

$$J_0 = -\frac{zyl}{2}\cos 2A$$

$$J_{45} = -\frac{zyl}{2}\sin 2A \;\;;$$

wherein the device is further configured to determine target values of at least one spectacle lens depending on the result of the comparison either on the basis of the retained subset of the subjective refraction data or on the basis of the combined refraction data;

- a manufacturing device, which is adapted to produce at least one spectacle lens based on the determined target values.

**Revendications**

1. Procédé de fabrication d'un verre de lunettes comprenant les étapes consistant à:

- fournir (S1) des données subjectives d'un utilisateur de verres de lunettes, les données subjectives comprenant au moins des données subjectives de réfraction $P_{sub}$;
- fournir (S2) des données de réfraction objectives $P_{obj}$ de l'utilisateur de verres de lunettes;
- comparer (S3) au moins un sous-ensemble des données de réfraction subjectives avec au moins un sous-ensemble des données de réfraction objectives et déterminer un résultat de comparaison;
- ajuster au moins le sous-ensemble de la réfraction subjective de la réfraction objective en raison du résultat de la comparaison dans l'hypothèse où les satisfait du résultat de la comparaison au moins une condition de comparaison prédéterminée, sinon à maintenir au moins le sous-ensemble de la réfraction subjective, ladite condition de comparaison est que deux de paramètres de comparaison de la réfraction ont une certaine différence ou dépasse, dans lequel l'ajustement du sous-ensemble de la réfraction subjective de la réfraction objective comprend le calcul de $P_K$ de réfraction combinée, et la réfraction combinée est calculée en additionnant la $P_K$ réfraction objective $P_{obj}$ avec la réfraction subjective $P_{sub}$ suit:

$$P_K = G_{obj}\, P_{obj} + G_{sub}\, P_{sub}$$

ou:

$$G = \begin{pmatrix} g_1 & 0 & 0 \\ 0 & g_2 & 0 \\ 0 & 0 & g_3 \end{pmatrix},$$

$$0 < g_i < 1,$$

$$G_{obj} + G_{sub} = 1$$

et dans lequel:

$$P = \begin{pmatrix} M \\ J_0 \\ J_{45} \end{pmatrix}$$

et

$$M = sph + \frac{zyl}{2}$$

$$J_0 = -\frac{zyl}{2}\cos 2A$$

$$J_{45} = -\frac{zyl}{2}\sin 2A \quad ;$$

- déterminer (S4) des valeurs de consigne au moins un des verres de lunettes, dans lequel les valeurs cibles sont déterminées en fonction du résultat de la comparaison, soit sur la base de la partie conservée de la réfraction subjective, ou sur la base de la réfraction combinée;
- produire au moins un verre de lunette en fonction de points de consigne spécifiques.

2. Procédé selon la revendication 1 avec l'étape suivante:
Fournir un message contenant le résultat de la comparaison.

3. Procédé selon la revendication 1 ou 2, dans lequel une pluralité de données de réfraction objectives peut être fourni et des données de réfraction objectives effectives peuvent être calculées sur la base de la pluralité de données de réfraction objectives, et dans lequel les données de réfraction objectives effectives sont comparée avec au moins le sous-ensemble des données subjectives de réfraction.

4. Procédé selon la revendication 3, **caractérisé en ce que** les données de réfraction objective effectives sont déterminées en tenant compte des conditions aux limites suivantes:

   - luminosité et / ou
   - diamètre de la pupille et / ou
   - contraste et / ou
   - l'âge.

5. Procédé selon l'une quelconque des revendications 3 et 4, dans lequel pour calculer la réfraction objective efficace d'une aberration, notamment en ordre variable, et / ou un OTF et / ou d'un MTF et / ou un PSF et / ou l'écart-type est / sont utilisés.

6. Procédé selon l'une quelconque des revendications précédentes, dans lequel au moins un sous-ensemble des données de réfraction objective est déterminé au moyen d'une photoréfraction excentrique.

7. Procédé selon l'une quelconque des revendications précédentes, dans lequel
$g_{sub,i} \geq 0,5$, ou en particulier
$g_{sub,1} \geq 0,6$ $g_{sub,2} \geq 0,5$ $g_{sub,3} \geq 0,7$ et $Abs(g_{sub,2} - g_{sub,3}) \leq 0,3$, dans lequel en outre, en particulier:
$g_{sub,1} = 0,7$ $g_{sub,2} = 0,6$ $g_{sub,3} = 0,8$ $Abs(g_{sub,2} - g_{sub,3}) \leq 0,1$.

8. Procédé selon l'une quelconque des revendications précédentes, dans lequel un équivalent sphérique M des données de réfraction objective est déterminé binoculaire, où:

$$M^{\bullet}_{r,obj} := M_{r,obj} - \frac{Dif}{2}$$

$$M^{\bullet}_{l,obj} := M_{l,obj} + \frac{Dif}{2}'$$

dans lequel

$$M_{Dif} = M_r - M_l$$

$$Dif = M_{Dif,obj} - (g_{obj\_bino} M_{Dif,obj} + g_{sub\_bino} M_{Dif,subj})$$

$$g_{obj\_bino} = 1 - g_{sub\_bino}$$

$$0 < g_{sub\_bino} < 1$$

9. Procédé selon la revendication 8, dans lequel:
$g_{sub\_bino} \geq 0,6$, ou en particulier :

$$g_{sub\_bino} = 0,9.$$

10. Produit de programme informatique qui, lorsqu'il est chargé dans la mémoire d'un ordinateur et exécuté par un ordinateur, oblige l'ordinateur à exécuter un procédé selon l'une quelconque des revendications précédentes, à l'exception de la dernière étape du procédé de la revendication 1.

11. Dispositif de fabrication d'un verre de lunettes avec:

   - un dispositif fournissant des données subjectives, conçu pour fournir des données subjectives d'un utilisateur de verres de lunettes, les données subjectives comprenant au moins des données de réfraction subjectives $P_{sub}$;
- un dispositif fournissant des données objectives, conçu pour fournir des données objectives de réfraction $P_{obj}$ de l'utilisateur de verres de lunettes;
- un dispositif de comparaison conçu pour comparer au moins un sous-ensemble des données de réfraction subjectives à au moins un sous-ensemble des données de réfraction objectives et conçu pour déterminer un résultat de comparaison;
- un dispositif d'adaptation qui est conçu pour adapter au moins le sous-ensemble de la réfraction subjective de la réfraction objective en raison du résultat de la comparaison dans l'hypothèse où les satisfait du résultat de la comparaison au moins une condition de comparaison prédéterminée, et dans lequel le moyen de réglage est adapté pour maintenir par ailleurs au moins le sous-ensemble de la réfraction subjective, dans lequel la condition de comparaison est que deux paramètres à comparer à partir des données réfractaires ont ou dépassent une certaine différence;

dans lequel, ajuster le sous-ensemble des données de réfraction subjectives aux données de réfraction objectives comprend le calcul de données de réfraction combinées $P_K$, et dans lequel les données de réfraction combinées $P_K$ sont calculées en ajoutant les données de réfraction objectives $P_{obj}$ aux données de réfraction subjectives $P_{sub}$ de la manière suivante:

$$P_K = G \cdot P_{obj} + G \cdot P_{sub}$$

ou:

$$G = \begin{pmatrix} g_1 & 0 & 0 \\ 0 & g_2 & 0 \\ 0 & 0 & g_3 \end{pmatrix},$$

$$0 < g_i < 1,$$

$$G_{obj} + G_{sub} = 1$$

et dans lequel:

$$P = \begin{pmatrix} M \\ J_0 \\ J_{45} \end{pmatrix}$$

et

$$M = sph + \frac{zyl}{2}$$

$$J_0 = -\frac{zyl}{2}\cos 2A$$

$$J_{45} = -\frac{zyl}{2}\sin 2A \quad ;$$

dans lequel le dispositif est en outre configuré pour déterminer des valeurs cibles d'au moins un verre de lunette en fonction du résultat de la comparaison, soit sur la base du sous-ensemble retenu des données de réfraction subjectives, soit sur la base des données de réfraction combinées;

- un dispositif de fabrication conçu pour produire au moins un verre de lunettes sur la base des valeurs nominales déterminées.

Figur 1

S1

| Subjektive Refraktionsdaten |

| Objektive Refraktionsdaten | S2

S3

| Plausibilisierung der subjektiven Refraktionsdaten aufgrund der objektiven Daten |

| Berechnung einer idealen Refraktion bzgl. MTF, RMS etc. aus den objektiven Daten | S2'

| Berechnung der Sollwerte für das Brillenglas aus der objektiven und subjektiven Refraktion | S4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20020140902 A1

- WO 2005058136 A2

### In der Beschreibung aufgeführte Nicht-Patentliteratur

- Principles of Optics. Born und Wolf. Pergamon Press, 1980, 203 ff, , 459 ff
- **GOODMAN.** Introduction to Fourier Optics. Mc-Graw-Hill, 1968

- **SMITH/ATCHISON.** The eye and visual optical instruments. Cambridge University Press, 1996